(19) Europäisches Patentamt
European Patent Office
Office européen des brevets

(11) Veröffentlichungsnummer: **0 059 980**
**A1**

(12) # EUROPÄISCHE PATENTANMELDUNG

(21) Anmeldenummer: **82101899.1**

(22) Anmeldetag: **10.03.82**

(51) Int. Cl.³: **A 01 N 37/12**
A 61 L 2/18, G 02 C 13/00
A 61 K 31/23

(30) Priorität: **11.03.81 DE 3109188**

(43) Veröffentlichungstag der Anmeldung:
**15.09.82 Patentblatt 82/37**

(84) Benannte Vertragsstaaten:
**AT BE CH FR GB IT LI LU NL SE**

(71) Anmelder: **NATEC Institut für naturwissenschaftlich-technische Dienste GmbH**
**Behringstrasse 154**
**D-2000 Hamburg 50(DE)**

(72) Erfinder: **vom Bruck, Carl Gerhard, Dr., Dipl.-Chem.**
**Klövensteenweg 97**
**D-2000 Hamburg 56(DE)**

(72) Erfinder: **Polzhofer, Kurt, Dr., Dipl.-Chem.**
**Bellmannstrasse 8**
**D-2000 Hamburg 52(DE)**

(74) Vertreter: **UEXKÜLL & STOLBERG Patentanwälte**
**Beselerstrasse 4**
**D-2000 Hamburg 52(DE)**

(54) **Verwendung eines antimikrobiell wirksamen Konservierungsmittels in Ophthalmica und Pflegemitteln für Kontaktlinsen.**

(57) Die Erfindung betrifft die Verwendung eines antimikrobiell wirksamen Konservierungsmittels in Ophthalmica und Pflegemitteln für Kontaktlinsen, das durch einen Gehalt an einem wasser- und alkohollöslichen Laurinsäurecholinestersalz charakterisiert ist und sich dadurch auszeichnet, daß es eine hervorragende bakterizide und fungizide Wirkung besitzt, gleichzeitig aber sehr gut verträglich ist und daher mit Vorteil in Ophthalmica und Pflegemitteln für Kontaktlinsen verwendet werden kann. Im allgemeinen kann der Wirkstoff in Konzentrationen von 0,02 bis 2,0 Gew.%, bezogen auf das Gesamtvolumen des Konservierungsmittels, angewendet werden.

EP 0 059 980 A1

Croydon Printing Company Ltd.

UEXKÜLL & STOLBERG
PATENTANWALTE

BESELERSTRASSE 4
D-2000 HAMBURG 52

EUROPEAN PATENT ATTORNEYS

DR. J.-D. FRHR. von UEXKULL
DR ULRICH GRAF STOLBERG
DIPL.-ING. JÜRGEN SUCHANTKE
DIPL.-ING. ARNULF HUBER
DR. ALLARD von KAMEKE
DR. KARL-HEINZ SCHULMEYER

0059930

NATEC - Institut für
naturwissenschaftlich-
technische Dienste GmbH

Behringstraße 154
2000 Hamburg 50

Prio: 11. März 1981

P 31 09 188.1-DE-

(18 531 sy/do)

März 1982

# Verwendung eines antimikrobiell wirksamen Konservierungsmittels in Ophthalmica und Pflegemitteln für Kontaktlinsen

Die Erfindung betrifft die Verwendung eines antimikrobiell wirksamen Konservierungsmittels in Ophthalmica und Pflegemitteln für Kontaktlinsen, das sich durch eine besonders intensive und breit gefächerte Wirksamkeit gegenüber grampositiven und gramnegativen Bakterien und Pilzen auszeichnet und gleichzeitig die hohen Ansprüche erfüllen kann, die auf den genannten Gebieten nicht nur an die antimikrobielle Wirksamkeit, sondern auch an die Verträglichkeit auf den menschlichen Organismus gestellt werden müssen.

Es ist bekannt, daß bestimmte quaternäre Ammoniumverbindungen nicht nur als Kationtenside, sondern auch als bakterizide und fungizide Additive eingesetzt werden können. So werden wasser- und alkohollösliche kationische Alkyltrimethylammoniumchloride, deren Alkylgruppen eine Kettenlänge von 8 bis 18 C-Atomen aufweisen, zum Beispiel unter dem Handelsnamen "Arquads" unter anderem in Desinfektionsmitteln verwendet. Aus DE-OS 16 42 070 werden als weitere wasserlösliche kationische quaternäre Ammoniumverbindungen Dequaliniumchlorid, Cetylpyridiniumchlorid und Benzalkoniumchlorid beschrieben, die auch in Gegenwart von Seifenresten ihre bakterizide Wirkung beibehalten sollen. Aus DE-OS 27 52 318 sind quaternäre Ammoniumderivate des Adamantans bekannt, die eine antibakterielle und fungizide Aktivität zeigen, darunter auch Cholinester mit einer 1-Adamantylgruppierung, also einer Kohlenwasserstoffgruppe, die einen dem Diamantgitter ähnlichen Gitteraufbau besitzt.

Obwohl demnach eine ganze Reihe von quaternären Ammoniumverbindungen bereits wegen ihrer bakteriziden und fungiziden Wirkungen zur Verwendung in Desinfektionsmitteln vorgeschlagen worden sind, hat sich gezeigt, daß der größte Teil dieser Klasse von Substanzen wegen ihrer Toxizität und/oder wegen ihrer Reizwirkung bei lokaler Applikation nur beschränkt anwendbar ist und insbesondere auf Gebieten, auf denen besonders hohe Anforderungen

an die Verträglichkeit und schonende Behandlung des betreffenden Organs gestellt werden, wie zum Beispiel in der Augenheilkunde, aus den genannten Gründen nicht verwendet werden können.

Der vorliegenden Erfindung liegt daher die Aufgabe zugrunde, einen Wirkstoff zu finden, mit dem die vorstehend genannten Nachteile ganz oder weitgehend vermieden werden können, und der insbesondere eine wirksame konservierende bzw. desinfizierende Wirkung bei sehr guter Verträglichkeit auch in Fällen zeigt, in denen an die Anwendung hinsichtlich einer schonenden, reizlosen und dennoch wirksamen Behandlung besondere Anforderungen gestellt werden.

Diese Aufgabe wird erfindungsgemäß gelöst durch die Verwendung von wasser- und alkohollöslichen Laurinsäurecholinestersalzen als antimikrobiell wirksame Konservierungsmittel in Ophthalmica und Pflegemitteln für Kontaktlinsen.

Die Laurinsäurecholinestersalze sind an sich bereits bekannt, zum Beispiel aus der DE-OS 2 157 290. Sie können nach bekannten Verfahren hergestellt werden, zum Beispiel durch Veresterung der Laurinsäure mit überschüssigem Dimethylaminoethanol bei 185°C, anschließende Quaternierung des gebildeten Laurinsäure-ß-dimethylaminoethylesters mit Methylchlorid bei etwa 45°C in einer geschlossenen Apparatur und anschließendes Umkristallisieren des erhaltenen Laurinsäurecholinesterchlorids aus Aceton mit 2 % Ethanol. Die Laurinsäurecholinestersalze sind kristalline, weiße Substanzen, die sich in Ethanol und Wasser gut lösen. Vorzugsweise werden das entsprechende Chlorid, Nitrat, Phosphat und/oder Acetat für den erfindungsgemäßen Zweck eingesetzt.

Die Laurinsäurecholinestersalze gehören in die homologe Reihe der Cholinestersalze der allgemeinen Formel

$$\left[R-\overset{O}{\overset{\|}{C}}-OCH_2CH_2\overset{+}{N}(CH_3)_3\right] X^-$$

in der R einen Alkylrest im allgemeinen mit 5 bis 17

C-Atomen und $X^-$ ein einwertiges Anion darstellt, wobei für die erfindungsgemäß vorgeschlagenen Salze R = 11 und $X^-$ = Chlorid-, Nitrat-, Phosphat- und/oder Acetatanion bedeuten. Wegen ihrer außerordentlich geringen Toxizität gelten diese Cholinestersalze als physiologisch unbedenklich und sind vom Bundesgesundheitsamt für den Oberflächenauftrag auf Lebensmittelverpackungen zugelassen.

Ein Gebiet, auf dem an die antimikrobielle Wirksamkeit und physiologische Verträglichkeit besonders hohe Anforderungen gestellt werden müssen, ist das der Kontaktlinsen-Pflegemittel und der Augenheilmittel. Eine wichtige Voraussetzung für eine problemlose Benutzung und Handhabung von Kontaktlinsen besteht in einer möglichst schonenden und dennoch wirksamen Pflege, wobei das oder die Pflegemittel einfach und sicher in ihrer Anwendung sein sollten. Neben einer milden, aber vollständigen Reinigungswirkung soll das Pflegemittel schonend desinfizierend wirken und auch bei häufiger Wiederholung des Reinigungsprozesses keine nachteiligen Veränderungen auf der Oberfläche der Kontaktlinsen verursachen. Ferner

soll das angewendete Pflegemittel in den angewandten Konzentrationen möglichst wenig toxisch und physiologisch gut verträglich sein, damit keine Irritationen oder andere Beeinträchtigungen am Auge auftreten können, durch die das Infektionsrisiko erhöht werden könnte.

Die tägliche Desinfektion der Kontaktlinsen ist eine Grundvoraussetzung für eine reibungslose Langzeitbenutzung. Die Kontaktlinsen werden während der tragefreien Zeit normalerweise in Kontaktlinsen-Pflegemittellösungen aufbewahrt und desinfiziert. Daher muß bei der Auswahl des geeigneten Desinfektions- und Konservierungsmittels eine besondere Sorgfalt angewandt werden.

Wegen der Möglichkeit, daß an den frisch desinfizierten, ins Auge eingesetzten Kontaktlinsen noch geringfügige Reste aus der Aufbewahrungslösung adsorbiert sind, die dann mit dem Auge bzw. mit der Tränenflüssigkeit des Auges in Berührung kommen, sind an die Beschaffenheit der Aufbewahrungslösung und speziell an die Eigenschaften der Konservierungs- bzw. Desinfektionsmittel in den Pflegemittellösungen strenge Anforderungen gestellt. Sie müssen in den angewandten Konzentrationen praktisch untoxisch für den menschlichen Organismus sein, dürfen keine Irritationen am Auge auslösen und sollen möglichst keine Ablagerungen auf den behandelten Kontaktlinsen

hinterlassen, sollen aber ein breites Wirkungsspektrum gegenüber Mikroorganismen haben.

Aufgrund dieser Anforderungen ist die Zahl der für diesen Zweck geeigneten Konservierungsstoffe sehr klein. An quaternären Ammoniumverbindungen haben sich in diesem Zusammenhang lediglich Benzalkoniumchlorid und Alkyltriethanolammoniumchloride als brauchbar erwiesen.

Es wurde nun überraschenderweise gefunden, daß unter den vorstehend mit einer allgemeinen Formel bezeichneten Cholinestersalzen die homologen wasser- und alkohollöslichen Laurinsäurecholinestersalze, insbesondere das Chlorid, den oben genannten Anforderungen in hohem Maße entsprechen und dabei im Vergleich zu bekannten quaternären Ammoniumverbindungen bei einer hervorragenden physiologischen Verträglichkeit eine unerwartet hohe antimikrobielle Wirksamkeit zeigen. Die erfindungsgemäßen Wirkstoffe sind daher als Konservierungsmittel bereits in sehr geringen Konzentrationen wirksam. Vorzugsweise wird der erfindungsgemäß vorgeschlagene Wirkstoff in einer Konzentration von etwa 0,02 bis 2,0 Gew.%, bezogen auf das Gesamtvolumen des Konservierungsmittels, angewendet.

Die antimikrobiellen Wirkungsspektren eines erfindungsgemäß vorgeschlagenen Wirkstoffes, nämlich des Laurinsäurecholinesterchlorids, und im Vergleich dazu von den homologen Essigsäure-, Capronsäure- und Palmitinsäurecholinesterchloriden sowie von den bekannten Konservierungsmitteln Adamantylcarbonsäurecholinesterchlorid und Chlorhexidin-diacetat wurden durch Bestimmung der minimalen Hemmkonzentration nach der Trübungsmethode in Pepton-Nährlösung bei einem pH von 7,0 festgestellt. Diese Methode ist von K.H. Wallhäußer und H. Schmidt in "Sterilisation, Desinfektion, Konservierung, Chemotherapie", Stuttgart (1967), Seite 420-421 beschrieben. Die Ergebnisse sind in Tabelle 1 zusammengestellt.

Aus der Tabelle 1 ist zu erkennen, daß der erfindungsgemäß verwendete Wirkstoff ein breites antimikrobielles Wirkungsspektrum besitzt, das mit dem des bekannten Konservierungsmittels Chlorhexidin-diacetat vergleichbar ist. Dieses Ergebnis ist besonders überraschend, da die zum Vergleich geprüften vier homologen Cholinesterchloride eine erheblich schlechtere antimikrobielle Wirksamkeit zeigen. Es konnte nicht vorhergesehen werden, daß das erfindungsgemäß vorgeschlagene Laurinsäurecholinestersalz eine um Größenordnungen bessere Wirksamkeit besitzt als die entsprechenden Homologen dieser Reihe. Wie aus Tabelle 1

Tabelle 1

Bestimmung der minimalen Hemmkonzentration (MIC-Werte) nach der Trübungsmethode in Pepton-Nährlösung bei pH 7,0

MIC-Werte in ppm

| Stämme | Laurinsäure-cholinester-chlorid | Essig-säure- | Capron-säure- | Palmitin-säure- | Adamantyl-carbonsäure-cholinester-chlorid | Chlor-hexidin-diacetat |
|---|---|---|---|---|---|---|
| | | cholinesterchlorid | | | | |
| Aspergillus fumigatus | 10 | >400 | >400 | 100 | >400 | 12,5 |
| Fusorium oxysporum | 3,13 | >400 | >400 | >400 | >400 | 12,5 |
| Paecilomyces variati | 0,78 | - | - | - | - | 1,56 |
| Paecilomyces Ehrlich | 0,78 | - | - | - | - | 0,78 |
| Candida albicans ATCC 10231 | 10 | >400 | >400 | 100 | >400 | 3,13 |
| Bacillus subtilis ATCC 6633 | 0,78 | - | - | - | - | 0,78 |
| Bacillus cereus ATCC 11778 | 3,13 | - | - | - | - | 0,78 |
| Staphylococcus aureus ATCC 25923 | 2,5 | >400 | >400 | 2,5 | 200 | 1,56 |
| Staphylococcus epidermis ATCC 12228 | 0,78 | - | - | - | - | 0,78 |
| Streptococcus pyogenes | 0,16 | 100 | 100 | 1,25 | 20 | 0,78 |
| E. coli ATCC 11229 | 10 | >400 | >400 | >400 | >400 | 3,13 |
| Enterobacter aerogenes | 100 | - | - | - | - | 12,5 |
| Proteus vulgaris | 200 | - | - | - | - | 50 |
| Pseudomonas fluorescens | 2,5 | - | - | - | - | - |
| Pseudomonas aeruginosa | 20 | >400 | >400 | >400 | >400 | - |

deutlich hervorgeht, ist das erfindungsgemäß vorgesehene Laurinsäurecholinestersalz hervorragend wirksam gegen grampositive und gramnegative Bakterien, Hefen und Pilze und erfüllt damit die an ein Konservierungsmittel gestellten Anforderungen in hohem Maße.

Anhand des Laurinsäurecholinesterchlorids als Beispiel für die erfindungsgemäß verwendeten Laurinsäurecholinestersalze wurde ferner eine akute orale Toxizitätsprüfung durchgeführt, wobei schwarze Mäuse als Versuchstiere dienten. Dabei wurde gefunden, daß der orale LD 50-Wert im Mittel bei 3,15 (2,66 bis 3,74) g/kg Tiergewicht liegt.

Bei der subakuten oralen Toxizitätsprüfung an Ratten konnten keine Wirkungen bei der Applikation von 20 und 50 mg Laurinsäurecholinesterchlorid pro kg Tiergewicht beobachtet werden.

Ferner wurde die Haut- und Schleimhautverträglichkeit von Laurinsäurecholinesterchlorid an Albino-Meerschweinchen aus einem leicht allergisierbaren Stamm (Pirlbright white) von 400 bis 700 g Gewicht und bei kleinwüchsigen Kaninchen von 1,5 bis 3 kg Gewicht geprüft. Die Tiere erhielten Standarddiät und Wasser nach Belieben. Die Raumtemperatur betrug etwa 20°C, die Luftfeuchtigkeit

etwa 50 %, die tägliche Belichtungsdauer 12 Stunden.

Die Prüfsubstanz wurde in einer Salbengrundlage aus Vaseline mit 200 ppm des Wirkstoffs auf direkte Hautreizung getestet. Bei 12 Meerschweinchen wurde die Präparation eine Woche lang täglich auf einem kurz geschorenen Bezirk an der Flanke aufgetragen und etwa 30 Sekunden eingerieben. Bei dieser Versuchsanordnung traten keine Hautreaktionen auf.

Daraufhin wurde bei den gleichen Tieren die lokale Anwendung der Prüfsubstanz auf drei Wochen ausgedehnt (Induktionsphase). Nach anschließender 5-tägiger Pause wurde die Prüfsubstanz an drei aufeinanderfolgenden Tagen auf einem nicht vorbehandelten kontralateralen kurzgeschorenen Kontrollbezirk eingerieben (Provokationsphase). Bei der Kontrolle nach 24 Stunden sowie nach 2 und 3 Tagen zeigte sich im Kontrollbezirk in keinem Falle eine Reaktion.

Aufgrund dieser Versuche ergab sich kein Hinweis auf sensibilisierende Eigenschaften des Laurinsäurecholinesterchlorids.

Die Prüfung auf Schleimhautverträglichkeit wurde am Kaninchenauge vorgenommen. Die Prüfsubstanz wurde als

Lösung von 2.000 ppm Laurinsäurecholinesterchlorid in 0,9 %iger wäßriger Kochsalzlösung im okkulären Reizwirkungstest nach Draize an acht Kaninchen auf Schleimhautverträglichkeit in der folgenden Weise geprüft.

0,1 ml der Testlösung wurden im Konjunktivalsack eines Tierauges unter Schließung von Ober- und Unterlid eingebracht. Man ließ die Lösung eine Minute lang einwirken. Das andere Auge wurde zur Kontrolle in gleicher Weise mit destilliertem Wasser behandelt.

Eine Minute nach der Applikation wurden bei der Hälfte der Tiere die behandelten Augen eine Minute lang mit 10 bis 20 ml lauwarmer physiologischer Kochsalzlösung nachgespült. Die Augen wurden anschließend bei allen behandelten Tieren geprüft. In keinem Falle kam es zu einer Reaktion im Sinne von Rötung, Schwellung, Sekretion oder Hornhautveränderung. Es wurden auch keine spontanen Abwehrreaktionen beobachtet.

Die vorstehenden Untersuchungen ergaben, daß Laurinsäurecholinesterchlorid einwandfrei haut- und schleimhautverträglich und frei von sensibilisierenden Eigenschaften ist.

Pharmazeutische Produkte in Form von Lösungen, die zur Kategorie I (Injektionspräparate und Ophthalmica) gehören,

müssen steril abgegeben werden. Da bei wiederholter Entnahme ein erhöhtes Kontaminationsrisiko besteht, müssen den Mehrfachentnahmebehältern Konservierungsmittel zugesetzt werden, damit die bei einer Entnahme eingebrachten Keime abgetötet werden können. Für die Beurteilung eines Konservierungsmittels ist es daher wichtig, in welcher Zeit alle eingebrachten Keime abgetötet werden. Hierüber können Konservierungsbelastungstests Auskunft geben.

Als Beispiel für die erfindungsgemäß vorgesehenen Konservierungsmittel wurde Laurinsäurecholinesterchlorid einem Konservierungsbelastungstest nach der Vorschrift USP XIX unterworfen und mit bekannten Konservierungsmitteln, die in Kontaktlinsen-Pflegelösungen eingesetzt werden und dem gleichen Test unterworfen wurden, verglichen. Die USP XIX schreibt vor, daß bei einem entsprechenden Belastungstest, bei dem man von einer Ausgangskeimzahl von $10^6$/ml ausgeht, eine Verminderung der Keimzahl innerhalb von 14 Tagen um 99,9 % bei den zugesetzten Bakterien erreicht wird und daß sich die zugesetzten Pilzsporen und Hefen innerhalb der gleichen Zeit nicht signifikant vermehren.

Um die antimikrobielle Wirkung der verschiedenen untersuchten Konservierungsmittel besser vergleichen zu können,

wurden die Belastungstest bei jeweils gleicher Wirkstoffkonzentration (0,01 % in steriler wäßriger Lösung) und bei gleicher Ausgangsbelastung des jeweils eingesetzten Stammes durchgeführt. In den Tabellen 2 bis 5 sind die Keimzahlen nach jeweils 2 und 10 Minuten sowie nach 1 Stunde, 2 und 24 Stunden angegeben, in den Tabellen 6 bis 9 sind die Ergebnisse nach 1 Tag, 7, 14, 21 und 28 Tagen festgehalten, da die Keimzahlen während der weiteren Laufzeit bis zum 28. Tag bei guter Konservierung nicht mehr ansteigen dürfen.

Die Tabellen 2 bis 9 zeigen deutlich, daß sich das erfindungsgemäß vorgesehene Laurinsäurecholinestersalz als Konservierungsmittel hervorragend eignet. Es ist in seiner antimikrobiellen Wirksamkeit mindestens vergleichbar mit den bekannten Konservierungsmitteln Benzalkoniumchlorid und Chlorhexidin-diacetat und übertrifft deutlich die zeitlich verzögerte Wirkung des ebenfalls als Konservierungsmittel bekannten Chlorbutols.

Wie aus der Monographie von K.H. Wallhäußer, Sterilisation, Desinfektion, Konservierung, 2. Auflage, Stuttgart (1978), Seite 390/391 hervorgeht, liegt eine ausreichende Konservierung vor, wenn die jeweilige Ausgangskeimzahl von $10^5$/g Probe bei Produkten der Kategorie I bei den Bakterien innerhalb eines Tages um zwei Zehnerpotenzen

Tabelle 2: *Konservierungsmittel - Belastungstest*: Laurinsäurecholinesterchlorid, Konzentr.: 0,01 % in dest. Wasser; pH: 7,0

| Stamm | Ausgangs-belastung | 2 Minuten | 10 Minuten | 1 Stunde | 2 Stunden | 24 Stunden |
|---|---|---|---|---|---|---|
| *Escherichia coli* | 9,5 · $10^6$ | 8,4 · $10^2$<br>7 · $10^1$ | 4 · $10^1$<br>— | —<br>— | —<br>— | —<br>— |
| *Pseudomonas aeruginosa* | 3,1 · $10^7$ | 2,2 · $10^4$<br>9,5 · $10^3$ | 9,1 · $10^2$<br>6,5 · $10^2$ | —<br>— | —<br>— | —<br>— |
| *Proteus vulgaris* | 2,1 · $10^7$ | 4,7 · $10^6$<br>2,8 · $10^6$ | 3,1 · $10^3$<br>6,2 · $10^3$ | 2<br>— | —<br>— | —<br>— |
| *Staphylococcus aureus* | 1,6 · $10^7$ | 1,4 · $10^7$<br>1,0 · $10^7$ | 9,1 · $10^4$<br>7,2 · $10^4$ | —<br>— | —<br>— | —<br>— |
| *Bacillus subtilis* | 6,8 · $10^5$ | 5,5 · $10^5$<br>3,8 · $10^5$ | 1,3 · $10^5$<br>1,4 · $10^5$ | 1,2 · $10^5$<br>1,3 · $10^5$ | 1,4 · $10^5$<br>1,2 · $10^5$ | 1,3 · $10^5$<br>7,6 · $10^2$ |
| *Candida albicans* | 2,3 · $10^6$ | 5,1 · $10^5$<br>3,7 · $10^5$ | 1,3 · $10^3$<br>7,1 · $10^2$ | —<br>— | —<br>— | —<br>— |
| *Aspergillus niger* | 1,6 · $10^5$ | 4,4 · $10^4$<br>4,2 · $10^4$ | 1,7 · $10^4$<br>2,1 · $10^4$ | 2,3 · $10^2$<br>2,2 · $10^2$ | 10<br>— | 20<br>— |
| *Kontrolle* | | —<br>— | —<br>— | —<br>— | —<br>— | —<br>— |

Tabelle 3: Konservierungsmittel - Belastungstest BENZALKONIUMCHLORID Konzentr.: 0,01 % in dest. Wasser; pH = 7,0

| Stamm | Ausgangs-belastung | 2 Minuten | 10 Minuten | 1 Stunde | 2 Stunden | 24 Stunden |
|---|---|---|---|---|---|---|
| Escherichia coli | $9,5 \cdot 10^6$ | –<br>– | –<br>– | –<br>– | –<br>– | –<br>– |
| Pseudomonas aeruginosa | $3,1 \cdot 10^7$ | 70<br>– | –<br>– | –<br>– | –<br>– | –<br>– |
| Proteus vulgaris | $2,1 \cdot 10^7$ | 30<br>– | –<br>– | –<br>– | –<br>– | –<br>– |
| Staphylococcus aureus | $1,6 \cdot 10^7$ | 130<br>80 | –<br>– | –<br>– | –<br>– | –<br>– |
| Bacillus subtilis | $6,8 \cdot 10^5$ | $4,9 \cdot 10^5$<br>$4,9 \cdot 10^5$ | $4,8 \cdot 10^4$<br>$4,5 \cdot 10^4$ | $1,2 \cdot 10^5$<br>$9,7 \cdot 10^4$ | $1,1 \cdot 10^5$<br>$8,4 \cdot 10^4$ | $4,1 \cdot 10^3$<br>$3,6 \cdot 10^3$ |
| Candida albicans | $2,3 \cdot 10^6$ | –<br>– | –<br>– | –<br>– | –<br>– | –<br>– |
| Aspergillus niger | $1,6 \cdot 10^5$ | $5,2 \cdot 10^3$<br>$2,3 \cdot 10^3$ | $4,4 \cdot 10^2$<br>$2,9 \cdot 10^2$ | 50<br>30 | 90<br>– | 90<br>– |
| Kontrolle | | –<br>– | –<br>– | –<br>– | –<br>– | –<br>– |

Tabelle 4: Konservierungsmittel - Belastungstest    CHLORHEXIDIN - DIACETAT    Konzentr.: 0,01 % in dest. Wasser; pH = 7,0

| Stamm | Ausgangs-belastung | 2 Minuten | 10 Minuten | 1 Stunde | 2 Stunden | 24 Stunden |
|---|---|---|---|---|---|---|
| Escherichia coli | $2{,}1 \cdot 10^7$ | $2{,}9 \cdot 10^3$<br>$4 \cdot 10^1$ | —<br>— | —<br>— | —<br>— | —<br>— |
| Pseudomonas aeruginosa | $2{,}9 \cdot 10^7$ | $9 \cdot 10^1$<br>$2{,}2 \cdot 10^2$ | —<br>— | —<br>— | —<br>— | —<br>— |
| Proteus vulgaris | $1{,}9 \cdot 10^7$ | $5{,}4 \cdot 10^3$<br>$4{,}9 \cdot 10^2$ | —<br>— | —<br>— | —<br>— | —<br>— |
| Staphylococcus aureus | $2{,}3 \cdot 10^7$ | $3{,}1 \cdot 10^2$<br>$5{,}2 \cdot 10^2$ | —<br>— | —<br>— | —<br>— | —<br>— |
| Bacillus subtilis | $5{,}7 \cdot 10^5$ | $5{,}2 \cdot 10^5$<br>$5{,}2 \cdot 10^5$ | $9{,}1 \cdot 10^4$<br>$1{,}4 \cdot 10^5$ | $2{,}8 \cdot 10^5$<br>$3{,}1 \cdot 10^5$ | $2{,}7 \cdot 10^5$<br>$2{,}9 \cdot 10^5$ | $1{,}3 \cdot 10^4$<br>$1{,}5 \cdot 10^4$ |
| Candida albicans | $2{,}4 \cdot 10^6$ | $5{,}1 \cdot 10^2$<br>— | —<br>— | —<br>— | —<br>— | —<br>— |
| Aspergillus niger | $1{,}6 \cdot 10^5$ | $1{,}6 \cdot 10^5$<br>$1{,}4 \cdot 10^5$ | $4{,}4 \cdot 10^5$<br>$4{,}6 \cdot 10^5$ | $5{,}1 \cdot 10^4$<br>$4{,}9 \cdot 10^4$ | $1{,}6 \cdot 10^5$<br>$1{,}4 \cdot 10^5$ | $2{,}3 \cdot 10^3$<br>$4{,}0 \cdot 10^3$ |
| Kontrolle | | —<br>— | —<br>— | —<br>— | —<br>— | —<br>— |

Tabelle 5: Konservierungsmittel - Belastungstest: CHLORBUTOL Konzentr.: 0,01 % in dest. Wasser; pH = 7,0

| Stamm | Ausgangs-belastung | 2 Minuten | 10 Minuten | 1 Stunde | 2 Stunden | 24 Stunden |
|---|---|---|---|---|---|---|
| Escherichia coli | $1{,}3 \cdot 10^7$ | $6{,}8 \cdot 10^6$<br>$8{,}7 \cdot 10^6$ | ·<br>· | ·<br>· | ·<br>· | ·<br>· |
| Pseudomonas aeruginosa | $8{,}2 \cdot 10^6$ | $8{,}7 \cdot 10^6$<br>$1{,}2 \cdot 10^7$ | $8{,}8 \cdot 10^6$<br>$8{,}3 \cdot 10^6$ | $9{,}1 \cdot 10^6$<br>$8{,}4 \cdot 10^6$ | $6 \cdot 10^6$<br>$1{,}3 \cdot 10^7$ | $5{,}5 \cdot 10^6$<br>$8{,}9 \cdot 10^6$ |
| Proteus vulgaris | $5 \cdot 10^7$ | $3{,}6 \cdot 10^7$<br>$4{,}7 \cdot 10^7$ | $3{,}4 \cdot 10^6$<br>$2{,}1 \cdot 10^6$ | $4{,}1 \cdot 10^6$<br>$1{,}8 \cdot 10^6$ | $6{,}7 \cdot 10^6$<br>$5{,}8 \cdot 10^6$ | $7{,}1 \cdot 10^6$<br>$6{,}2 \cdot 10^6$ |
| Staphylococcus aureus | $9 \cdot 10^6$ | $9{,}1 \cdot 10^6$<br>$8{,}4 \cdot 10^6$ | $1{,}4 \cdot 10^6$<br>$2{,}8 \cdot 10^6$ | $1{,}3 \cdot 10^7$<br>$7{,}4 \cdot 10^6$ | ·<br>· | $6{,}6 \cdot 10^6$<br>$8{,}7 \cdot 10^6$ |
| Bacillus subtilis | $1 \cdot 10^5$ | $6{,}2 \cdot 10^4$<br>$4{,}8 \cdot 10^4$ | $3{,}8 \cdot 10^4$<br>$2{,}2 \cdot 10^4$ | $5{,}3 \cdot 10^4$<br>$3{,}7 \cdot 10^4$ | ·<br>· | $3{,}9 \cdot 10^4$<br>$2{,}4 \cdot 10^4$ |
| Candida albicans | $1{,}3 \cdot 10^7$ | $9{,}5 \cdot 10^6$<br>$9{,}7 \cdot 10^6$ | $3{,}8 \cdot 10^5$<br>$4{,}1 \cdot 10^5$ | $8{,}1 \cdot 10^3$<br>$9{,}3 \cdot 10^3$ | $8{,}4 \cdot 10^3$<br>$7{,}9 \cdot 10^2$ | $6{,}1 \cdot 10^3$<br>$9{,}2 \cdot 10^2$ |
| Aspergillus niger | $1{,}7 \cdot 10^5$ | $1{,}1 \cdot 10^5$<br>$6 \cdot 10^4$ | $3{,}3 \cdot 10^4$<br>$3{,}6 \cdot 10^4$ | $5{,}7 \cdot 10^4$<br>$6{,}4 \cdot 10^4$ | $4{,}6 \cdot 10^4$<br>$6{,}1 \cdot 10^4$ | $3{,}8 \cdot 10^2$<br>$3{,}1 \cdot 10^2$ |
| Kontrolle | · | –<br>– | –<br>– | –<br>– | –<br>– | –<br>– |

Tabelle 6

Konservierungsmittel - Belastungstest (nach USP 19)

Laurinsäurecholinesterchlorid, Konzentr.: 0,01 % in dest. Wasser; pH = 7,0

| Stamm<br>Ausgangs-<br>belastung | 1. Tag | 7. Tag | 14. Tag | 21. Tag | 28. Tag |
|---|---|---|---|---|---|
| Escherichia coli<br>$84.10^7$ | -<br>-<br>-<br>-<br>- | -<br>-<br>-<br>-<br>- | -<br>-<br>-<br>-<br>- | -<br>-<br>-<br>-<br>- | -<br>-<br>-<br>-<br>- |
| Pseudomonas aeruginosa<br>$93.10^7$ | -<br>-<br>-<br>-<br>- | -<br>-<br>-<br>-<br>- | -<br>-<br>-<br>-<br>- | -<br>-<br>-<br>-<br>- | -<br>-<br>-<br>-<br>- |
| Staphylokokkus aureus<br>$85.10^7$ | -<br>-<br>-<br>-<br>- | -<br>-<br>-<br>-<br>- | -<br>-<br>-<br>-<br>- | -<br>-<br>-<br>-<br>- | -<br>-<br>-<br>-<br>- |
| Candida albicans<br>$56.10^7$ | -<br>-<br>-<br>-<br>- | -<br>-<br>-<br>-<br>- | -<br>-<br>-<br>-<br>- | -<br>-<br>-<br>-<br>- | -<br>-<br>-<br>-<br>- |
| Aspergillus niger<br>$56.10^5$ | -<br>-<br>-<br>-<br>- | -<br>-<br>-<br>-<br>- | -<br>-<br>-<br>-<br>- | -<br>-<br>-<br>-<br>- | -<br>-<br>-<br>-<br>- |
| Kontrolle | -<br>-<br>-<br>-<br>- | -<br>-<br>-<br>-<br>- | -<br>-<br>-<br>-<br>- | -<br>-<br>-<br>-<br>- | -<br>-<br>-<br>-<br>- |

Tabelle 7

Konservierungsmittel - Belastungstest (nach USP 19)

Benzalkoniumchlorid (Fa. Merck), Konzentr.: 0,01 % in dest. Wasser; pH = 7,0

| Stamm Ausgangs-belastung | 1. Tag | 7. Tag | 14. Tag | 21. Tag | 28. Tag |
|---|---|---|---|---|---|
| Escherichia coli $84.10^7$ | - - - - - | - - - - - | - - - - - | - - - - - | - - - - - |
| Pseudomonas aeruginosa $93.10^7$ | - - - - - | - - - - - | - - - - - | - - - - - | - - - - - |
| Staphylokokkus aureus $85.10^7$ | - - - - - | - - - - - | - - - - - | - - - - - | - - - - - |
| Candida albicans $56.10^7$ | - - - - - | - - - - - | - - - - - | - - - - - | - - - - - |
| Aspergillus niger $56.10^5$ | - - - - - | - - - - - | - - - - - | - - - - - | - - - - - |
| Kontrolle | - - - - - | - - - - - | - - - - - | - - - - - | - - - - - |

Tabelle 8

Konservierungsmittel - Belastungstest (nach USP 19)

Chlorhexidin-Diacetat (Serva), Konzentr.: 0,01 % in dest. Wasser; pH = 7,0

| Stamm Ausgangs-belastung | 1. Tag | 7. Tag | 14. Tag | 21. Tag | 28. Tag |
|---|---|---|---|---|---|
| Escherichia coli $84.10^7$ | - | - | - | - | - |
| | - | - | - | - | - |
| | - | - | - | - | - |
| | - | - | - | - | - |
| | - | - | - | - | - |
| Pseudomonas aeruginosa $93.10^7$ | - | - | - | - | - |
| | - | - | - | - | - |
| | - | - | - | - | - |
| | - | - | - | - | - |
| | - | - | - | - | - |
| Staphylokokkus aureus $85.10^7$ | - | - | - | - | - |
| | - | - | - | - | - |
| | - | - | - | - | - |
| | - | - | - | - | - |
| | - | - | - | - | - |
| Candida albicans $56.10^7$ | - | - | - | - | - |
| | - | - | - | - | - |
| | - | - | - | - | - |
| | - | - | - | - | - |
| | - | - | - | - | - |
| Aspergillus niger $56.10^5$ | 670 | 230 | 40 | 10 | - |
| | 540 | 140 | 30 | - | - |
| | 570 | 310 | 20 | - | - |
| | 730 | 250 | 110 | 10 | - |
| | 570 | 200 | 100 | 10 | - |
| Kontrolle | - | - | - | - | - |
| | - | - | - | - | - |
| | - | - | - | - | - |
| | - | - | - | - | - |
| | - | - | - | - | - |

Tabelle 9

Konservierungsmittel - Belastungstest (nach USP 19)

Chlorbutol (Fa. Merck), Konzentr.: 0,01 % in dest. Wasser; pH = 7,0

| Stamm Ausgangs-belastung | 1. Tag | 7. Tag | 14. Tag | 21. Tag | 28. Tag |
|---|---|---|---|---|---|
| Escherichia | $140.10^{4}$ | $154.10^{3}$ | 14 | - | - |
| coli | $121.10^{4}$ | $141.10^{3}$ | 6 | - | - |
| $84.10^{7}$ | $117.10^{4}$ | $71.10^{3}$ | - | - | - |
| | $117.10^{4}$ | $312.10^{3}$ | 80 | - | - |
| | $123.10^{4}$ | $128.10^{3}$ | - | - | - |
| Pseudomonas | $77.10^{4}$ | 250 | - | - | - |
| aeruginosa | $73.10^{4}$ | - | - | - | - |
| $93.10^{7}$ | $59.10^{4}$ | 200 | - | - | - |
| | $61.10^{4}$ | - | - | - | - |
| | $75.10^{4}$ | - | - | - | - |
| Staphylokokkus | $82.10^{4}$ | - | - | - | - |
| aureus | $71.10^{4}$ | - | - | - | - |
| $85.10^{7}$ | $72.10^{4}$ | - | - | - | - |
| | $67.10^{4}$ | - | - | - | - |
| | $63.10^{4}$ | - | - | - | - |
| Candida | $58.10^{4}$ | $138.10^{2}$ | 10 | - | - |
| albicans | $33.10^{4}$ | $48.10^{1}$ | - | - | - |
| $56.10^{7}$ | $44.10^{4}$ | $145.10^{1}$ | - | - | - |
| | $40.10^{4}$ | $46.10^{1}$ | - | - | - |
| | $39.10^{4}$ | $67.10^{1}$ | 10 | - | - |
| Aspergillus | 190 | 330 | 260 | 90 | - |
| niger | 140 | 310 | 240 | 60 | - |
| $56.10^{5}$ | 160 | 290 | 120 | 20 | - |
| | 170 | 320 | 170 | 20 | - |
| | 140 | 320 | 230 | 10 | - |
| Kontrolle | - | - | - | - | - |
| | - | - | - | - | - |
| | - | - | - | - | - |
| | - | - | - | - | - |
| | - | - | - | - | - |

und nach zwei Wochen um drei Zehnerpotenzen abfällt und während der weiteren Laufzeit (28 Tage) nicht mehr ansteigt und Hefen und Schimmelpilze nach zwei Wochen um wenigstens zwei Zehnerpotenzen zurückgehen und später nicht mehr ansteigen. Diese Bedingungen werden von dem erfindungsgemäß verwendeten Laurinsäurecholinesterchlorid weit übertroffen.

Die erfindungsgemäß vorgeschlagenen Laurinsäurecholinestersalze besitzen auch die erforderliche Stabilität und Beständigkeit. So wurde eine 5 %ige wäßrige Laurinsäurecholinesterchloridlösung 8 Stunden am Sieden gehalten. Danach wurde eine nur unwesentliche Zunahme des Säuregehalts der Trockensubstanz von 2,5 auf 3,9 % festgestellt. Demnach kann unter den Bedingungen, unter denen die erfindungsgemäß vorgeschlagenen Wirkstoffe normalerweise als Konservierungsmittel eingesetzt werden, eine hydrolytische Spaltung des Wirkstoffes praktisch ausgeschlossen werden.

Ferner wurde eine 1 %ige wäßrige Laurinsäurecholinesterchloridlösung in Gegenwart von Pankreassaft simulierten physiologischen Bedingungen (38°C) ausgesetzt und nach 1 Stunde und nach 24 Stunden Proben gezogen und auf entstandene freie Fettsäuren untersucht. Dazu wurden die Proben mit Petroläther extrahiert und im Extrakt

die Fettsäuren mikroanalytisch bestimmt. Dabei ergab sich, daß nach 1 Stunde nur 1,5 %, nach 24 Stunden nur 2,5 % des Substrats verseift waren. Im Kontrollversuch ohne Pankreassaft wurde keine Hydrolyse festgestellt.

Das als Bezugssubstanz für die Enzymaktivität verwendete Glycerinmonocaprylat zeigte hingegen unter den gleichen Bedingungen eine Abbauquote von 13,8 %.

Die vorstehenden Untersuchungsergebnisse zeigen demnach, daß die erfindungsgemäß verwendeten antimikrobiellen Konservierungsmittel den bisher bekannten Konservierungsmitteln auf dem Gebiet der Kontaktlinsen-Pflegemittel mindestens ebenbürtig sind und daß sie sich durch eine hervorragende Haut- und Schleimhautverträglichkeit auszeichnen. Trotz ihrer sehr guten antimikrobiellen Wirksamkeit sind die erfindungsgemäß vorgesehenen Laurinsäurecholinestersalze, insbesondere das Chlorid, in den für die Applikation vorgesehenen Mengen völlig untoxisch und physiologisch unbedenklich. Dies bedeutet ein geringeres Risiko und damit einen wesentlichen Vorteil gegenüber vergleichbaren Konservierungsmitteln wie Benzalkoniumchlorid oder Chlorhexidinacetat.

Die erfindungsgemäß verwendeten Laurinsäurecholinestersalze sind daher mit Vorteil insbesondere wegen ihrer vorzüglichen Schleimhautverträglichkeit, als Konservierungsmittel in Ophthalmica und in Pflegemitteln für Kontaktlinsen geeignet. Außerdem sind sie natürlich auch zur Konservierung von kosmetischen Zubereitungen vorteilhaft einsetzbar, beispielsweise in Haarwässern, Hautcremes, Hautnähremulsionen, Shampoos und Frisiermitteln. Der Wirkstoff wird den Zubereitungen in einer Menge zugemischt, die eine zuverlässige konservierende Wirkung gewährleistet. Im allgemeinen sind hierbei Konzentrationen von 0,02 bis 2,0 Gew.%, bezogen auf die Gesamtmenge der Zubereitungen, ausreichend. Die Verwendungsmöglichkeiten des erfindungsgemäßen Konservierungsmittels werden anhand der nachfolgenden Beispiele weiter erläutert.

Beispiel 1

Herstellung einer Augentropfen-Suspension

Eine Augentropfen-Suspension wurde gemäß dem Formularium der Nederlandse Apothekers (F.N.A. 1968) wie folgt hergestellt:

30 mg Natriumdihydrogenphosphat, 850 mg Natriumchlorid und 20 mg Dinatriumhydrogenphosphat wurden in ca. 90 ml konserviertem Wasser gelöst. Das konservierte Wasser enthielt als Konservierungsmittel 0,02 % Laurinsäurecholinesterchlorid und 0,1 % Dinatriumethylendiamintetraacetat.

1,0 mg Hydrocortisonacetatund 2,5 mg Polyvidon wurden in einem Mörser sorgfältig miteinander vermischt, dann 0,5 ml der obengenannten Lösung zugefügt und weiter sorgfältig gemischt. Anschließend wurden nochmals 0,5 ml der Lösung zugefügt, gemischt, und der Rest der Lösung in Portionen weiter zugefügt. Dann wurde mit konserviertem Wasser auf 100 ml aufgefüllt und die Mischung durch ein G2-Glasfilter filtriert. Die filtrierte Mischung wurde 30 Minuten lang auf 100°C erhitzt. Die erhaltene Suspension wurde ins Tiefkühlfach eines Kühlschrankes gestellt, bis sie gefroren war. Bei Zimmertemperatur ließ man sie unter kräftigem Schütteln auftauen. Anschließend wurde die Mischung unter aseptischen Bedingungen in Augentropfenfläschchen von 10 ml Inhalt abgefüllt.

## Beispiel 2

### Herstellung einer Benetzungsflüssigkeit für Kontaktlinsen

Formulierung:

| | |
|---|---|
| Natriumchlorid | 0,45 g |
| Kaliumchlorid | 0,37 g |
| Hydroxypropylmethylzellulose (4500 cP) | 0,3 g |
| Borax | 0,19 g |
| Borsäure | 0,19 g |
| Laurinsäurecholinesterchlorid | 0,02 g |
| destilliertes Wasser | auf 100 ml auffüllen |

Beispiel 3

Herstellung einer Aufbewahrungsflüssigkeit für weiche Kontaktlinsen

Formulierung:

| | |
|---|---|
| Natriumchlorid | 0,9 g |
| EDTA (Komplexon III) | 0,1 g |
| Laurinsäurecholinesterchlorid | 0,5 g |
| destilliertes Wasser | auf 100 ml auffüllen |

Beispiel 4

Herstellung einer Aufbewahrungslösung für harte und weiche Kontaktlinsen

Formulierung:

| | |
|---|---|
| Laurinsäurecholinesterchlorid | 1,00 g |
| Dinatrium-EDTA | 1,00 g |
| $NaH_2PO_4 \cdot H_2O$ | 5,10 g |
| $Na_2HPO_4$ | 14,80 g |
| Kollidon 25 | 0,03 g |
| Tylopur MH 1000 | 2,90 g |
| Harnstoff | 60,00 g |
| bidestilliertes Wasser | auf 1 l auffüllen |

Die Aufbewahrungslösung besaß einen pH-Wert von 7,3.

0059980

## Beispiel 5

Abspüllösung für harte und weiche Kontaktlinsen

Formulierung:

| | |
|---|---|
| Laurinsäurecholinesterchlorid | 0,2 g |
| Dinatrium-EDTA | 1,00 g |
| Natriumchlorid | 9,00 g |
| Natriumhydrogencarbonat | 0,50 g |
| bidestilliertes Wasser | auf 1 l auffüllen |

Die Abspüllösung besaß einen pH-Wert von 7,5.

## Beispiel 6

Reinigungslösung für harte und weiche Kontaktlinsen

Formulierung:

| | |
|---|---|
| Dinatrium-EDTA | 1,00 g |
| $NaH_2PO_4 \cdot H_2O$ | 5,10 g |
| $Na_2HPO_4$ | 14,80 g |
| Laurinsäurecholinesterchlorid | 0,50 g |
| Tween 40 | 0,10 g |
| Kollidon 25 | 0,03 g |
| Tylopur MH 1000 | 5,80 g |
| bidestilliertes Wasser | auf 1 l auffüllen |

Die Reinigungslösung besaß einen pH-Wert von 7,3.

## Patentanspruch

Verwendung von wasser- und alkohollöslichen Laurinsäurecholinestersalzen als antimikrobiell wirksame Konservierungsmittel in Ophthalmica und Pflegemitteln für Kontaktlinsen.

0059980

Nummer der Anmeldung

EP 82 10 1899

Europäisches Patentamt

## EUROPÄISCHER RECHERCHENBERICHT

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| Y | CHEMICAL ABSTRACTS, Band 76, 1972, Zusammenfassung 61261b, Seite 96<br>COLUMBUS, OHIO (US)<br>& IZV.VYSSH.UCHEB.ZAVED.KHIM. KHIM.TEKHNOL. 1971, 14(9)1369-73<br>I.P.KOMKOV u.a.: "Surface-active quaternary ammonium salts of o-acylcholines"<br><br>* die ganze Zusammenfassung * | <br><br><br><br><br><br>1 | A 01 N 37/12<br>A 61 L 2/18<br>G 02 C 13/00<br>A 61 K 31/23 |
| | -- | | RECHERCHIERTE SACHGEBIETE (Int. Cl.3) |
| Y | US - A - 3 639 576 (H.H.KASPAR u.a.)<br><br>* Spalte 3, Zeilen 16-24; Patentanspruch 1 * | <br><br>1 | A 61 L<br>A 01 N<br>G 02 C<br>A 61 K |
| | --------------- | | |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| DEN HAAG | 18. Mai 1982 | PELTRE |